# EUROPÄISCHE PATENTSCHRIFT

(11) **EP 0 639 367 B1**
(45) Veröffentlichungstag und Bekanntmachung des Hinweises auf die Patenterteilung: **19.08.1998**
(21) Anmeldenummer: 94109370.0
(22) Anmeldetag: 17.06.1994
(51) Int. Cl.: A61K 6/04

(54) **Verwendung von Gold-Palladiumlegierungen für Dentalgussteile**
Use of gold-palladium alloys in dental restorations
Utilisation d'alliages d'or et palladium pour des restaurations dentaires

(30) Priorität: 23.07.1993 DE 4324738
(43) Veröffentlichungstag der Anmeldung: 22.02.1995
(73) Patentinhaber: Degussa Aktiengesellschaft, 60311 Frankfurt (DE)
(72) Erfinder: Kempf, Bernd, Dr. Dipl.-Ing., D-63579 Freigericht (DE); Hathaway, Doris, D-63456 Hanau (DE); Schöck, Gernot, Dipl.-Ing., D-63486 Bruchköbel (DE); Ringelstein, Hans-Martin, D-60358 Frankfurt/M. (DE)

(56) Entgegenhaltungen:
- CH-A- 488 017
- DE-B- 2 851 729
- US-A- 3 666 540
- US-A- 4 205 982
- DATABASE WPI Section Ch, Week 7335 Derwent Publications Ltd., London, GB; Class D21, AN 73-49327U & JP-A-47 038 827 (MITSUBISHI MET IND KK) , 7.Dezember 1972

## Beschreibung

Die Erfindung betrifft die Verwendung von hochgoldhaltigen Gold-Palladiumlegierungen für mit Keramik verblendete und unverblendete Dentalgußteile.

Festsitzender und herausnehmbarer Zahnersatz wird häufig aus korrosionsbeständigen, biokompatiblen Edelmetallegierungen hergestellt, wobei das gegossene Objekt anschließend oft mit Dentalkeramik verblendet wird, um ein dem natürlichen Zahn entsprechendes Aussehen zu erzielen. Die Eignung von Legierungen für diesen Zweck ist an eine Reihe von Eigenschaften geknüpft, die auf die Dentalkeramik abgestimmt sein müssen, wie thermischer Ausdehnungskoeffizient, Schmelzintervall und Haftung zwischen Keramik und Legierung. Grundvoraussetzung ist auch eine gute Korrosionsbeständigkeit und eine ausreichende Festigkeit, um die Belastungen beim Kauvorgang zu ertragen. Nach ihrer mechanischen Belastbarkeit werden Dentallegierungen in verschiedenen Klassen von Typ 1 bis 4 eingeteilt. Die höchste Festigkeit und damit die breiteste Indikation besitzen Typ 4-Legierungen.

Hochgoldhaltige Edelmetallegierungen sind die traditionellen Legierungssysteme, die für diesen Zweck eingesetzt werden.

Sie haben sich seit vielen Jahren klinisch bewährt. In der Korrosionsbeständigkeit und Biokompatibilität sind diese Legierungen nach wie vor unerreicht. Die zahlreichen, oben erwähnten Anforderungen, die an diese Legierungen gestellt werden, konnten bisher aber nur mit in aller Regel sehr kompliziert aufgebauten Legierungssystemen erfüllt werden.

Die hochgoldhaltigen Aufbrennlegierungen sind charakterisiert durch einen Goldgehalt ab ca. 70 Gew.%. Zur Steigerung der Hochtemperaturstabilität während des Keramikbrandes ist in der Regel Palladium und Platin zulegiert. Da Platin das Schmelzintervall einer Goldlegierung wesentlich stärker aufweitet als Palladium, lassen sich besonders hochtemperaturstabile Legierungen speziell durch Palladium als Legierungselement erhalten. Solche Legierungen besitzen in der Regel Palladiumgehalte von mindestens 6 Gew.%. Zur Erhöhung der Härte und der mechanischen Festigkeit sind eine Reihe verschiedener Nichtedelmetalle zulegiert. Weitere Elemente werden zulegiert, um die Feinabstimmung weiterer zahntechnisch relevanter Daten, wie thermischer Ausdehnungskoeffizient, Keramikhaftung, Oxidfarbe oder ausreichende Duktilität bei hoher Temperatur zu gewährleisten. Gebräuchliche weitere Legierungselemente sind daher Silber, Kupfer, Indium, Zink, Zinn, Eisen und Gallium. Bekannt ist, daß eine Reihe dieser Elemente auch wieder unerwünschte Eigenschaften haben kann, so daß versucht wird, diese zu vermeiden oder nur in geringer Menge einzusetzen. Beispielsweise kann Silber zu Grünverfärbung bei empfindlichen Keramiken führen und Kupfer kann speziell beim Auftreten von Spaltkorrosionseffekten zu Verfärbungen führen.

Bekannte hochgoldhaltige Dentallegierungen enthalten meist zwei oder mehr Unedelmetallegierungselemente, um alle für einen Zahnersatz notwendigen Legierungseigenschaften einzustellen.

In der US-PS 3,716,356 wird eine hochgoldhaltige Dentallegierung beschrieben, die 5,5 bis 40 Gew.% Palladium und 0,03 bis 1,0 Gew.% Rhenium enthält. Außerdem können noch bis zu 10 Gew.% Platin, bis zu 2 Gew.% Silber, bis zu 1 Gew.% Eisen, bis zu 1,5 Gew.% Zink, bis zu 2 Gew.% Zinn und bis zu 1 Gew.% Indium anwesend sein. Härtewerte sind für diese Legierungen nicht angegeben, doch erreichen sie die Härte von Typ 4-Legierungen nur bei Anwesenheit weiterer Unedelmetallbestandteile, über deren toxische Wirkungen noch wenig bekannt ist.

Hochgoldhaltige Dentallegierungen gemäß DE-OS 30 19 276 enthalten neben Palladium bis zu 10 Gew.% Indium und außerdem Ruthenium und Zinn, um ausreichende Härtewerte zu erzielen.

Die DE-PS 24 24 575 beschreibt hochgoldhaltige Dentallegierungen, die 5 bis 15 Gew.% Platin, 0,1 bis 2 Gew.% Indium, 0,05 bis 0,5 Gew.% Iridium und 0,5 bis 3 Gew.% Rhodium enthält. Diese Legierungen sind palladiumfrei.

Die in der Praxis verwendeten hochgoldhaltigen Dentallegierungen von Typ 4 enthalten alle zwei oder mehr Unedelmetallkomponenten, um entsprechende Härtewerte zu erreichen.

Im Zuge eines allgemein gestiegenen Gesundheitsbewußtseins und einer generell zu beobachtenden höheren Anfälligkeit gegenüber Allergien und Unverträglichkeiten bei den Menschen der modernen Industriestaaten ist auch die Biokompatibilität von Dentallegierungen verstärkt in die Diskussion geraten. Bisherige Untersuchungen zeigen, daß Art und Menge der durch Korrosionsvorgänge in Lösung gehenden Bestandteile einer Legierung für die Biokompatibilität entscheidend sind. Die Ursachen der Korrosion und die möglichen Wirkungen der Korrosionsprodukte auf den Organismus sind dabei sehr komplexer Natur. Untersuchungen deuten darauf hin, daß speziell die während des Keramikbrandes auftretende thermische Belastung und Oxidation der Aufbrennlegierungen ein wesentlicher Faktor ist, der die Korrosionsbeständigkeit der Legierungen herabsetzt. Anzustreben ist generell ein möglichst hoher Edelmetallanteil für gute Korrosionsbeständigkeit und eine möglichst geringe Anzahl der Legierungskomponenten, speziell der Nichtedelmetalle, um dadurch die Wahrscheinlichkeit einer allergischen Reaktion auf eine bestimmte Komponente so gering wie möglich zu halten. Selbstverständlich sollten nur Elemente Verwendung finden, von denen keinerlei toxische Wirkungen bekannt sind.

Es war daher Aufgabe der vorliegenden Erfindung, hochgoldhaltige Gold-Palladiumlegierungen für mit Keramik verblendete und unverblendete Dentalgußteile zu entwickeln, die zur Erzielungen einer für Typ 4-Legierungen notwendigen Härte nur ein einziges, in seiner toxischen Wirkung bekanntes Unedelmetall benötigen. Außerdem sollten diese Legierungen eine bessere Korrosionsbeständigkeit aufweisen als die bisherigen Legierungen und alle sonstigen für Aufbrennlegierungen notwendigen Eigenschaften besitzen, wie Festigkeit, Duktilität, Wärmeausdehnungskoeffizient, Keramikhaftung und Hochtemperaturstabilität.

Diese Aufgabe wird erfindungsgemäß dadurch gelöst, daß man Legierungen verwendet, die 6 bis 25 Gew.% Palladium, 0 bis 12 Gew.% Platin, 0 bis 2 Gew.% Iridium, Rhodium und/oder Ruthenium und 0,7 bis 5,8 Gew.% Zinn, Rest Gold, enthalten, wobei
a) der Zinngehalt bei Platingehalten unter 2 Gew.% in einem Bereich liegt, der im Palladium-Zinn-Diagramm durch die Punkte A, B, C und D begrenzt ist, mit A = 6 Pd, 1,3 Sn, B = 6 Pd, 2,8 Sn, C = 25 Pd, 5,8 Sn, D = 25 Pd 2,2 Sn,
b) der erlaubte Zinngehalt bei Platingehalten oberhalb 2 Gew.% pro 2 Gew.% Platingehalt um jeweils 0,12 Gew.% Zinn erniedrigt wird, und
c) die Summe der Gehalte von Palladium und Platin 30 Gew.% nicht überschreitet.

Vorzugsweise verwendet man Legierungen, die 12 bis 25 Gew.% Palladium, 0 bis 10 Gew.% Platin und 0 bis 2 Gew.% Iridium, Rhodium und/oder Ruthenium als Kornfeiner, 2,1 bis 5,0 Gew.% Zinn, Rest Gold enthalten und bei denen der Zinngehalt in einem Bereich liegt, der im Palladium-Zinn-Diagramm durch die Punkte A', B', C', und D begrenzt ist, mit A' = 12 Pd, 2,1 Sn, B' = 12 Pd, 3,0 Sn, C' = 25 Pd, 5,0 Sn und D mit der vorstehenden Bedeutung.

Besonders bewährt haben sich Legierungen, die im Palladium-Zinn-Diagramm durch Punkte A', B', C'' und D'' begrenzt werden, mit C'' = 16 Pd, 3,5 Sn , D'' = 16 Pd, 2,2 Sn , A' und B' mit der vorstehenden Bedeutung.

Bewährt haben sich auch Legierungen, die im Palladium-Zinn-Diagramm durch die Punkte A, B, C''' und D''' begrenzt werden, mit C''' = 10 Pd, 3,4 Sn D'''= 10 Pd, 1,5 Sn, A und B mit der vorstehenden Bedeutung, wobei die Summe von Palladium und Platin 12 Gew.% nicht übersteigen darf.

Überraschenderweise zeigt es sich, daß alle gestellten Anforderungen an Dentallegierungen erfüllt werden können von Legierungen, die als einziges Unedelmetall Zinn enthalten. Diese Legierungen besitzen eine exzellente Korrosionsbeständigkeit, die deutlich über der Korrosionsbeständigkeit der heute bekannten Legierungen liegt. Voraussetzung für diese Eigenschaften ist, daß der Zinngehalt dieser Legierungen in definierter Weise an den Palladiumgehalt angepaßt wird und bei Anwesenheit von Platin auch noch in bezug auf den Platingehalt modifiziert wird. Diese Legierungen besitzen aufgrund der sehr guten Korrosionsbeständigkeit und der Tatsache, daß die Unbedenklichkeit des Unedelmetalls Zinn durch seine vielfältige Verwendung in der Nahrungsmittelindustrie als Zinngeschirr oder als Weißblech belegt ist, eine außergewöhnliche Biokompatibilität.

Legierungen dieses Systems bestehen im einfachsten Fall aus Gold, Palladium und Zinn und können darüber hinaus noch 0-2 % Iridium, Rhodium und/oder Ruthenium als Kornfeinungsmittel enthalten. Sehr gute Korrosionsbeständigkeiten und ausreichende Härten für Legierungen vom Typ 4 werden erreicht, wenn der Zinngehalt genau auf den Palladiumgehalt abgestimmt ist. Und zwar ist umso mehr Zinn nötig, je höher der Palladiumgehalt der Legierung liegt. Das mögliche Zinn/Palladiumverhältnis ist in Figur 1 dargestellt. Danach sind die zulässigen Zinngehalte beim zulässigen Palladiumgehalt von 6-25 % durch ein viereckiges Feld in einem Pd-Sn-Diagramm definiert, dessen Eckpunkte bei Pd-Gehalten von 6 % bei 1,3 und 2,8 % Zinn liegen und bei Pd-gehalten von 25 % zwischen 2,2 und 5,8 Zinn.

Die Zulegierung von Platin hat den Vorteil, daß die Anteile des Unedelmetalls Zinn weiter abgesenkt werden können. Die Korrosionsbeständigkeit und damit auch die Biokompatibilität wird dadurch noch weiter verbessert. Die Zulegierung von Platin ist auf 12 % begrenzt, wobei in der Summe nicht mehr als 30 Gew.% Palladium und Platin in der Legierung enthalten sein sollen. Ab einem Platin-Gehalt von 2 Gew.% aufwärts lassen sich die erforderlichen Zinngehalte im Schnitt um 0,12 Gew.% pro 2 Gew.% Platin reduzieren.

In Tabelle I sind eine Reihe von Legierungen nach ihrer Zusammensetzung aufgeführt. Die Legierungen 1-6 sind Legierungen, die dem Stand der Technik entsprechen. Sie enthalten mindestens zwei Unedelmetalle. Die Legierungen 7-12 stellen Versuchslegierungen dar, die zwar nur Zinn als Unedelmetall enthalten, bei denen der Zinngehalt aber außerhalb des in Fig. 1 skizzierten Bereiches liegt. Die Legierungen 13-20 entsprechen in ihrer Zusammensetzung den erfindungsgemäßen Anforderungen bzgl. Pd-, Sn- und Pt-Gehalt.

**Tabelle I**

| Legierungszusammensetzungen: | | | | | | | |
|---|---|---|---|---|---|---|---|
| Legierung | Au | Pd | Pt | Ag | Sn | In | Sonstige |
| 1 | 77,3 | 8,9 | 9,8 | <2,0 | <2,0 | <2,0 | Cu,Fe,Re,Ir |
| 2 | 84,4 | 5,0 | 8 | - | - | 2,5 | Ta |
| 3 | 72 | 9,7 | 13 | 2,8 | 1,2 | 1,2 | Ir |
| 4 | 74,8 | 15 | 6 | - | 2 | 2 | Ir |
| 5 | 86 | - | 10,4 | - | - | <2,0 | Rh,Ta |
| 6 | 77,7 | - | 19,5 | - | - | - | Zn,Ta |
| 7 | 64,9 | 25 | 4 | - | 6,0 | - | 0,1 Ru |
| 8 | 77,8 | 20 | - | - | 1,8 | - | 0,4 Ru |
| 9 | 83,3 | 15 | - | - | 1,5 | - | 0,2 Ir |
| 10 | 91 | 7,5 | - | - | 1,3 | - | 0,2 Ir |
| 11 | 88,8 | 7,5 | - | - | 3,5 | - | 0,2 Ir |
| 12 | 75,4 | 14 | 6 | - | 4,5 | - | 0,1 Ir |
| 13 | 72,8 | 23 | - | - | 4,0 | - | 0,2 Ir |
| 14 | 81,5 | 15 | - | - | 3,0 | - | 0,5 Ir |
| 15 | 77,8 | 14 | 6 | | 2,1 | - | 0,1 Ir |
| 16 | 81,8 | 14 | 2 | | 2,0 | - | 0,2 Ir |
| 17 | 76,8 | 13 | 8 | | 2,2 | - | |
| 18 | 86,8 | 10 | - | | 2,0 | - | 1,2 Rh |
| 19 | 89,1 | 7 | 2 | | 1,8 | | 0,1 Ir |
| 20 | 81,3 | 14,5 | - | | 4,0 | - | 0,2 Ir |

In Tabelle 2 sind die Ergebnisse von Korrosionsuntersuchungen zusammengestellt. Zur Bestimmung der Korrosionsbeständigkeit wurden Korrosionsversuche nach dem DIN-Entwurf 13927 durchgeführt. Dazu werden Probekörper für 7 Tage in einer 0,1m Milchsäure- 0,1 m Kochsalzlösung bei 37^{o} C gelagert. Anschließend wird die Korrosionslösung mittels geeigneter Analyseverfahren qualitativ und quantitativ auf die freigesetzten Korrosionsprodukte analysiert. Um Oberflächeneffekte und den Einfluß der Oxidation auszuschließen, werden die Prüfkörper nach einem zuvor simulierten Keramikbrand vor dem Einsetzen in die Korrosionslösung abgeschliffen. In den durchgeführten Untersuchungen wurden neben diesem "Standard-Korrosionstest" verschärfte Testbedingungen zusätzlich gewählt, um gerade den Einfluß der Oxidation auf das Korrosionsverhalten zu untersuchen. Dies ist notwendig, da davon auszugehen ist, daß unter realen Bedingungen nicht der gesamte Zahnersatz nach dem Keramikbrand so mechanisch nachbearbeitet werden kann, daß die vorausgehende oxidative Schädigung der unverblendeten Bereiche komplett entfernt wird. Zur Simulation dieser Bedingungen wurden ausgewählte Legierungen sandgestrahlt und oxidiert und ohne nachfolgende Entfernung der Oxidschicht in die Korrosionslösung gehängt.

In der Tabelle 2 sind die jeweils analysierten Konzentrationen der gelösten Legierungsbestandteile aufgeführt. In der letzen Spalte ist zusätzlich die Summe der Gesamtionenkonzentration aufgelistet, die das wesentliche Kriterium im DIN Entwurf 13927 darstellt. Und zwar darf die Summe aller gelösten Ionen nach 7 Tagen Korrosion nicht den Grenzwert von 100 µg/cm² überschreiten. Die Korrosionsraten der Elemente, die unterhalb der jeweiligen Nachweisgrenze liegen, werden in dem Summenwert nicht berücksichtigt. Die Nachweisgrenze liegt bei 0,13 µg/cm².

**Tabelle 2**

| Konzentrationen der gelösten Elemente in den Korrosionslösungen in µg/cm² | | | | | | | | |
|---|---|---|---|---|---|---|---|---|
| Leg.-Nr. | Au | Pd | Pt | Ag | Sn | In | Sonstige | Gesamtkonzentration |
| Standard-Korrosionstest | | | | | | | | |
| | | | | | | | | |
| 1 | <0,13 | <0,13 | <0,13 | <0,13 | <0,13 | 0,50 | | 0,50 |
| 2 | <0,04 | <0,13 | <0,13 | - | | 0,39 | | 0,39 |
| 4 | <0,13 | <0,13 | <0,13 | - | 0,14 | 0,62 | | 0,76 |
| 6 | <0,13 | | <0,13 | - | | | Zn: 20,8 | 20,8 |
| 13 | <0,13 | <0,13 | - | - | 0,17 | - | | 0,17 |
| 14 | <0,13 | <0,13 | <0,13 | - | <0,13 | - | | 0 |
| 15 | <0,13 | <0,13 | <0,13 | - | <0,13 | - | | 0 |
| 18 | <0,13 | <0,13 | <0,13 | - | <0,13 | - | | 0 |
| 19 | <0,13 | <0,13 | <0,13 | - | <0,13 | | | 0 |
| 20 | <0,13 | <0,13 | <0,13 | - | <0,13 | | | 0 |

| Verschärfte Testbedingungen: | | | | | | | | |
|---|---|---|---|---|---|---|---|---|
| Leg.-Nr. | Au | Pd | Pt | Ag | Sn | In | Sonstige | Gesamtkonzentration |
| 1 | <0,13 | <0,13 | 0,35 | 0,13 | 0,77 | 5,8 | Cu: 3,4; Fe: 0,47 | 10,8 |
| 2 | <0,13 | <0,13 | <0,13 | - | - | 8,79 | - | 8,79 |
| 4 | <0,13 | <0,13 | <0,13 | - | 1,4 | 6,2 | | 7,6 |
| 13 | <0,13 | <0,13 | <0,13 | - | 0,38 | | | 0,38 |
| 14 | <0,13 | <0,13 | <0,13 | - | 0,52 | - | - | 0,52 |
| 15 | <0,13 | <0,13 | <0,13 | - | <0,13 | - | | 0 |
| 19 | <0,13 | <0,13 | <0,13 | - | <0,13 | - | | 0 |
| 20 | <0,13 | <0,13 | <0,13 | - | <0,13 | - | - | 0 |

Die Ergebnisse belegen, daß die hochgoldhaltigen Aufbrennlegierungen allesamt Korrosionswerte besitzen, die weit unter den vorgeschriebenen Grenzwerten liegen. Es fällt jedoch auf, daß nur bei den erfindungsgemäßen Legierungen alle analysierten Elemente unterhalb oder knapp oberhalb der jeweiligen Nachweisgrenze liegen. Bei den verschärften Korrosionsbedingungen wird dieser Unterschied zwischen den Legierungen, die den Stand der Technik darstellen und den erfindungsgemäßen Legierungen noch deutlicher. Während die Legierungen nach dem Stand der Technik einen signifikanten Anstieg in den Korrosionsdaten aufweisen, zeigen die erfindungsgemäßen Legierungen auch unter diesen Bedingungen nur sehr geringe Korrosionsraten. Meist liegen die analysierten Elemente sogar unter der jeweiligen Nachweisgrenze.

Die herausragende Beständigkeit der erfindungsgemäßen Legierungen gegenüber Oxidation, die wohl für die gute Korrosionsbeständigkeit ursächlich ist, wird auch durch metallographische und thermogravimetrische Untersuchungen belegt. An metallographischen Schliffen lassen sich bei den Legierungen nach dem Stand der Technik ausgeprägte innere Oxidationszonen nachweisen, während bei den erfindungsgemäßen Legierungen die Oxidsäume so dünn sind, daß sie im Lichtmikroskop fast nicht zu erkennen sind. Durch die Oxidation der Legierungen kommt es zu einer Gewichtszunahme, die sich thermogravimetrisch bestimmen läßt. Dazu wurden aus einer Reihe von Legierungen dünne Ringe gegossen, um dem angreifenden Sauerstoff eine möglichst große Oberfläche zu bieten. Diese Ringe wurden an einer Thermowaage hängend mit einer definierten Aufheizgeschwindigkeit von 30 K/min auf 950^{o} C aufgeheizt, auf dieser Temperatur 120 min gehalten und anschließend wieder mit einer konstanten Abkühlrate von 30 K/min abgekühlt. Während des Versuches wurde die Gewichtszunahme kontinuierlich gemessen. In Tabelle 3 sind die Gesamtgewichtszunahmen, die ein Maß für die abgelaufene Oxidation darstellen, aufgeführt. Die erfindungsgemäßen Legierungen (Nr. 14, 15, 17, 19) zeichnen sich durch die niedrigsten Gewichtszunahmen aus.

**Tabelle 3**

| Gewichtszunahmen durch Oxidation | |
|---|---|
| Leg.-Nr. | Gewichtszunahme [mg/cm²] |
| | |
| 1 | 0,58 |
| 2 | 0,69 |
| 3 | 0,38 |
| 5 | 0,66 |
| 4 | 0,46 |
| 14 | 0,29 |
| 15 | 0,26 |
| 17 | 0,24 |
| 19 | 0,21 |

Zur Charakterisierung der Raumtemperaturfestigkeit sind in Tabelle 4 die Härtewerte nach dem Guß, im ausgehärteten Zustand und nach der Ofenreise aufgelistet, sowie die Streckgrenze, die Zugfestigkeit und die Bruchdehnung. Die Zugproben wurden nach DIN-Entwurf 13927 wärmebehandelt, so daß ein Gefüge vorlag, wie es nach dem Keramikbrand existiert.

**Tabelle 4**

| Härtewerte und mechanische Festigkeit. | | | | | | |
|---|---|---|---|---|---|---|
| Leg.-Nr. | HV-Guß | HV-hart | HV-Ofenr. | Rp[MPa] | Rm[MPa] | A[%] |
| 1 | 193 | 225 | 200 | 490 | 630 | 10,5 |
| 2 | 170 | 220 | 180 | 480 | 590 | 8,2 |
| 4 | 203 | 250 | 205 | 569 | 701 | 5,2 |
| 6 | 202 | 230 | 190 | | | |
| 7 | 210 | 235 | 225 | 573 | 638 | 1,8 |
| 8 | 86 | | 90 | | | |
| 9 | 115 | 128 | | | | |
| 10 | 125 | 127 | | | | |
| 11 | 185 | | 193 | 488 | 530 | 1,4 |
| 12 | 175 | | 187 | 518 | 543 | 1,9 |
| 13 | 206 | 228 | 216 | | | |
| 14 | 200 | 216 | 231 | 572 | 693 | 5,4 |
| 15 | 182 | 223 | 202 | 558 | 696 | 7,6 |
| 16 | 193 | | 196 | 534 | 673 | 6,1 |
| 17 | 178 | | 169 | | | |
| 18 | 194 | | 172 | 488 | 562 | 5,7 |
| 19 | 153 | | 164 | | | |
| 20 | 206 | | 190 | | | |

Die Versuchslegierungen (8, 9, 10) mit Zinngehalten die unterhalb des erfindungsgemäßen Bereiches liegen, zeigen nur geringe Härtewerte und Festigkeiten. Die Versuchslegierungen (Nr. 7, 11, 12), die zu hohe Zinngehalte aufweisen, besitzen zwar hohe Härtewerte und Zugfestigkeiten, aber ihre Duktilität ist zu gering. Wie metallographische Untersuchungen zeigen, ist die Ausbildung einer zweiten Phase dafür verantwortlich.

In Tabelle 5 sind die Messungen zur Hochtemperarturstabilität der Legierungen zusammengestellt. Die Hochtemperaturstabilität der Legierungen während des Keramikbrandes wird durch den sogenannten "sag resistance" d.h. den Widerstand gegen die Verformung bei hoher Temperatur aufgrund des Eigengewichtes, definiert. Zur Bestimmung des "sag resistance" wurden Probestäbchen der Dimension 50 mm x 3 mm x 1 mm durch Feinguß hergestellt und mittels Sandstrahlen von Einbettmasse gereinigt. Zur Simulation des Keramikbrandes wurden die Proben bei 980^{o} C einer 20-minütigen Wärmebehandlung ausgesetzt, wobei sie horizontal auf zwei Keramikstützen auf der Flachseite liegend, gelagert wurden. Die Keramik stützen besaßen einen Abstand von 40 mm, so daß die Möglichkeit bestand, daß sich die Proben unter ihrem Eigengewicht durchbiegen. Das Ausmaß der Verbiegung wurde ermittelt, indem die Probestäbchen vor und nach dem Keramikbrand mittels eines induktiven Wegeaufnehmersystems vermessen wurden. Der Unterschied in der Durchbiegung vor und nach dem Keramikbrand stellt ein Maß für die Hochtemperaturstabilität dar. Wie aus Tabelle 5 hervorgeht, zeigen die Legierungen ohne bzw. mit geringen Palladiumgehalten eine starke Durchbiegung (Leg. Nr. 5 und 6). Legierungen mit relativ hohen Palladiumgehalten sind wesentlich hochtemperaturstabiler, Besonders gute Hochtemperaturstabilitäten zeigen die Legierungen (Nr. 13, 15 und 17), mit Palladiumgehalten, die über 12 % liegen.

**Tabelle 5**

| Ergebnisse zum "sag resistance test" | |
|---|---|
| Leg.-Nr. | Durchbiegung in µm, (Mittelwerte) |
| 1 | 98 |
| 2 | 420 |
| 4 | 60 |
| 5 | 790 |
| 6 | 880 |
| 13 | 75 |
| 15 | 43 |
| 17 | 38 |
| 18 | 330 |
| 19 | 460 |

Legierungen mit Palladiumgehalten unter 12 % haben bei hohen Temperaturen nicht ganz so gute Festigkeitseigenschaften (Leg. Nr. 18 und 19), diese Legierungen besitzen aber den Vorteil, daß sie noch eine gelbe bzw. gelbliche Farbe aufweisen, die aus ästhetischen Gründen bevorzugt wird.

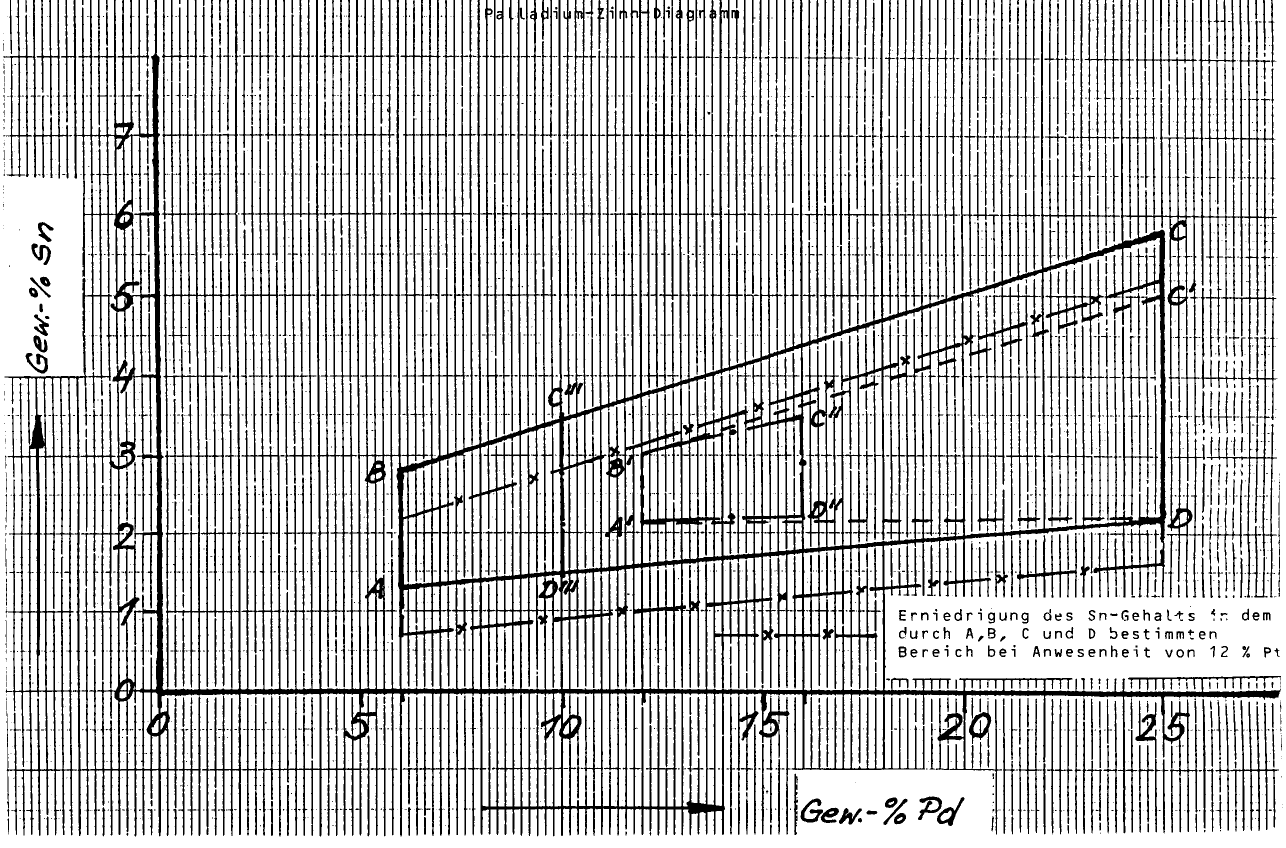

## Patentansprüche

1. Verwendung von hochgoldhaltigen Gold-Palladiumlegierungen, bestehend aus 6 bis 25 Gew.% Palladium, 0 bis 12 Gew.% Platin, 0 bis 2 Gew.% Iridium, Rhodium und/oder Ruthenium und 0,7 bis 5,8 Gew.% Zinn, Rest Gold, für mit Keramik verblendete und unverblendete Dentalgußteile, wobei
a) der Zinngehalt bei Platingehalten unterhalb 2 Gew.% in einem Bereich liegt, der im Palladium-Zinn-Diagramm durch die Punkte A, B, C und D begrenzt ist, mit A = 6 Pd 1,3 Sn, B = 6 Pd 2,8 Sn, C = 25 Pd 5,8 Sn und D = 25 Pd 2,2 Sn;
b) der erlaubte Zinngehalt bei Platingehalten oberhalb 2 Gew.% pro 2 Gew.% Platingehalt um jeweils 0,12 Gew.% Zinn erniedrigt wird, und
c) die Summe der Gehalte von Palladium und Platin 30 Gew.% nicht überschreitet.

2. Verwendung von hochgoldhaltigen Gold-Palladiumlegierungen nach Anspruch 1, bestehend aus 12 bis 25 Gew.% Palladium, 0 bis 10 Gew.% Platin, 0 bis 2 Gew.% Iridium, Rhodium und/oder Ruthenium, 2,1 bis 5,0 Gew.% Zinn, Rest Gold, wobei der Zinngehalt in einem Bereich liegt, der im Palladium-Zinn-Diagramm durch die Punkte A', B', C' und D begrenzt wird, mit A' = 12 Pd 2,1 Sn, B' = 12 Pd 3,0 Sn, C' = 25 Pd 5,0 Sn und D mit der vorstehenden Bedeutung, und Platinzusätze die gleiche Absenkung des Zinngehaltes bewirken wie in Anspruch 1.

3. Verwendung von hochgoldhaltigen Gold-Palladiumlegierungen nach Anspruch 1 und 2, die im Palladium-Zinn-Diagramm durch die Punkte A', B', C'' und D'' begrenzt werden, mit C'' = 16 Pd 3,5 Sn, D'' = 16 Pd 2,2 Sn, A' und B' mit der vorstehenden Bedeutung.

4. Verwendung von hochgoldhaltigen Gold-Palladiumlegierungen nach Anspruch 1, bestehend aus 6 bis 10 Gew.% Palladium, 0 bis 6 Gew.% Platin, 0 bis 2 Gew.% Iridium, Rhodium und/oder Ruthenium, 1,3 bis 3,4 Zinn, Rest Gold, wobei der Zinngehalt in einem Bereich liegt, der im Palladium-Zinn-Diagramm durch die Punkte A, B, C''' und D''' begrenzt wird, mit C''' = 10 Pd 3,4 Sn, D''' = 10 Pd 1,5 Sn, A und B mit der vorstehenden Bedeutung, und die Summe von Palladium und Platin 12 Gew.% nicht übersteigt.

## Claims

1. Use of gold/palladium alloys with an elevated gold content consisting of 6 to 25 wt.% of palladium, 0 to 12 wt.% of platinum, 0 to 2 wt.% of iridium, rhodium and/or ruthenium and 0.7 to 5.8 wt.% of tin, remainder gold, for dental castings with and without ceramic overlays, wherein,
a) at platinum contents of below 2 wt.%, the tin content is within a range delimited on the palladium/tin diagram by the points A, B, C and D, where A = 6 Pd 1.3 Sn, B = 6 Pd 2.8 Sn, C = 25 Pd 5.8 Sn and D = 25 Pd 2.2 Sn;
b) at platinum contents of above 2 wt.%, the permitted tin content is reduced by 0.12 wt.% of tin per 2 wt.% of platinum content, and
c) the sum of palladium and platinum contents does not exceed 30 wt.%.

2. Use of gold/palladium alloys with an elevated gold content according to claim 1 consisting of 12 to 25 wt.% of palladium, 0 to 10 wt.% of platinum, 0 to 2 wt.% of iridium, rhodium and/or ruthenium, 2.1 to 5.0 wt.% of tin, remainder gold, wherein the tin content is within a range delimited on the palladium/tin diagram by the points A', B', C' and D, where A' = 12 Pd 2.1 Sn, B' = 12 Pd 3.0 Sn, C' = 25 Pd 5.0 Sn and D has the above-stated meaning, and additions of platinum bring about the same reduction in tin content as in claim 1.

3. Use of gold/palladium alloys with an elevated gold content according to claims 1 and 2 which are delimited on the palladium/tin diagram by the points A', B', C'' and D'', where C'' = 16 Pd 3.5 Sn, D'' = 16 Pd 2.2 Sn, A' and B' have the above-stated meaning.

4. Use of gold/palladium alloys with an elevated gold content according to claim 1 consisting of 6 to 10 wt.% of palladium, 0 to 6 wt.% of platinum, 0 to 2 wt.% of iridium, rhodium and/or ruthenium, 1.3 to 3.4 [sic] tin, remainder gold, wherein the tin content is within a range delimited on the palladium/tin diagram by the points A, B, C''' and D''', where C''' = 10 Pd 3.4 Sn, D''' = 10 Pd 1.5 Sn, A and B have the above-stated meaning, and the sum of palladium and platinum does not exceed 12 wt.%.

## Revendications

1. Utilisation d'alliages d'or et de palladium à haute teneur en or, constitués de 6 à 25 % en poids de palladium, de 0 à 12 % en poids de platine, de 0 à 2 % en poids d'iridium, de rhodium et/ou de ruthénium et de 0,7 à 5,8 % en poids d'étain, le reste étant constitué d'or, pour des restaurations dentaires incrustées et non incrustées de céramique, dans lesquelles
a) la teneur en étain pour des teneurs en platine inférieures à 2 % en poids se situe dans une zone qui est limitée dans le diagramme palladium-étain par les points A, B, C et D, avec A = 6 Pd, 1,3 Sn, B = 6 Pd, 2,8 Sn, C = 25 Pd, 5,8 Sn, D = 25 Pd, 2,2 Sn ;
b) la teneur en étain permise pour des teneurs en platines supérieures à 2 % en poids pour 2 % en poids de teneur en platine est toujours abaissée de 0,12 % d'étain, et
c) la somme des teneurs en palladium et en platine ne dépasse pas 30 % en poids.

2. Utilisation d'alliages d'or et de palladium à haute teneur en or selon la revendication 1,
constituée de 12 à 25 % en poids de palladium, de 0 à 10 % en poids de platine et de 0 à 2 % en poids d'iridium, de rhodium et/ou de ruthénium, de 2,1 à 5,0 % en poids d'étain, le reste étant constitué d'or, la teneur en étain se situant dans une zone qui est limitée dans le diagramme palladium-étain par les points A', B', C' et D, avec A' = 12 Pd, 2,1 Sn, B' = 12 Pd, 3,0 Sn, C' = 25 Pd, 5,0 Sn et D ayant la signification donnée ci-dessus, et où les additions de platine provoquent le même abaissement de la teneur en étain que dans la revendication 1.

3. Utilisation d'alliages d'or et de palladium à haute teneur en or selon la revendication 1 et 2,
qui sont limités dans le diagramme palladium-étain par les points A', B', C'' et D'' avec C'' = 16 Pd, 3,5 Sn, D'' = 16 Pd, 2,2 Sn, A' et B' ayant la signification donnée ci-dessus.

4. Utilisation d'alliages d'or et de palladium à haute teneur en or selon la revendication 1,
constitués de 6 à 10 % en poids de palladium, 0 à 6 % en poids de platine, 0 à 2 % en poids d'iridium, de rhodium et/ou de ruthénium, 1,3 à 3,4 % en poids d'étain, le reste étant constitué d'or, la teneur en étain se situant dans une zone qui est limitée dans le diagramme palladium-étain par les points A, B, C''' et D''', avec C''' = 10 Pd, 3,4 Sn, D''' = 10 Pd, 1,5 Sn, A et B ayant la signification donnée ci-dessus, et la somme du palladium et du platine ne doit pas dépasser 12 % en poids.
